# EUROPEAN PATENT APPLICATION

(11) **EP 3 973 846 A1**
(43) Date of publication of application: **30.03.2022**
(21) Application number: 20808907.8
(22) Date of filing: 11.05.2020
(51) Int. Cl.: A61B 1/06, A61B 1/07, H01S 5/022

(54) **LUMINESCENCE DEVICE, AND ELECTRONIC APPARATUS AND INSPECTION METHOD USING SAID LUMINESCENCE DEVICE**

(30) Priority: 20.05.2019 JP 2019094624
(71) Applicant: Panasonic Intellectual Property Management Co., Ltd., Osaka-shi, Osaka 540-6207 (JP)
(72) Inventor: ABE Takeshi, Osaka-shi, Osaka 540-6207 (JP); NITTA Mitsuru, Osaka-shi, Osaka 540-6207 (JP); OSHIO Shozo, Osaka-shi, Osaka 540-6207 (JP)
(74) Representative: Müller-Boré & Partner Patentanwälte PartG mbB
(86) International application number: PCT/JP2020/018780
(87) International publication number: WO 2020/235369

(57) **Abstract**

A light emitting device (1, 1A) includes a solid-state light emitting element that emits a pulsed light, a wavelength converter (4, 4A) including a phosphor that emits a wavelength-converted light having a wavelength longer than that of the pulsed light, and a light guide member (3) including a light input end (3a) and a light output end (3b). The wavelength converter is disposed on a light output end side of the light guide member. The pulsed light is input to the light guide member through the light input end and output through the light output end to be emitted to the wavelength converter. The wavelength-converted light has a fluorescence spectrum having a maximum intensity value in a wavelength range exceeding 710 nm. An electronic apparatus includes the light emitting device. An inspection method includes using the light emitting device.

## Description

### TECHNICAL FIELD

The present invention relates to a light emitting device, and an electronic apparatus and an inspection method using the light emitting device.

### BACKGROUND ART

There has been known a white light emitting device including a wavelength conversion member at a light output end of a light guide member (see, Patent Literature 1). The light emitting device of Patent Literature 1 generates white light by wavelength conversion of excitation light using a wavelength conversion member including a visible light emitting phosphor. The light emitting device is described to be applied to an endoscope device for imaging the inside of a living body, by using a light guide member having a small diameter.

In contrast, a near-infrared light emitting device having an excitation light source and a wavelength conversion member has also been known (see, Patent Literature 2). The light emitting device of Patent Literature 2 generates near-infrared light by wavelength conversion of excitation light using the wavelength conversion member including a near-infrared light emitting phosphor. The light emitting device is described to be usable for a composition analyzing device or the like because it emits a wideband near-infrared light.

The near-infrared light emitting device is expected to be applied to a medical lighting. This is because the near-infrared light has a property of easily passing through a living body, and thus, it is advantageous for obtaining information inside the living body and treating lesions inside the living body. Specific examples of the medical lighting include a lighting for a fluorescence imaging method, and a lighting for a photodynamic therapy. The fluorescence imaging method and the photodynamic therapy are particularly expected to be applied to an endoscope, and thus it is desired to provide a near-infrared light emitting device suitable for an endoscopic application.

### CITATION LIST

### PATENT LITERATURE

Patent Literature 1: Japanese Unexamined Patent Application Publication No. 2005-205195
Patent Literature 2: Japanese Unexamined Patent Application Publication No. 2018-518046

### SUMMARY OF INVENTION

However, in a light emitting device simply having a near-infrared light emitting phosphor at the light output end of a light guide member, the temperature of the light output end rises due to the heat generated by the near-infrared light emitting phosphor having a large stokes loss. When the high temperature light output end touches a living body, the living body is damaged, and thus it is difficult to use it in the living body.

The present invention has been made in consideration of such an issue as described above, which is inherent in related art. An object of the present invention is to provide a light emitting device that emits a near-infrared light and is usable even in a temperature-limited environment, such as in a living body, and an electronic apparatus and an inspection method using the light emitting device.

To solve the above issue, a light emitting device according to a first aspect of the present invention includes: a solid-state light emitting element that emits a pulsed light; a wavelength converter including a phosphor that emits a wavelength-converted light having a wavelength longer than that of the pulsed light; and a light guide member including a light input end and a light output end. The wavelength converter is disposed on a light output end side of the light guide member. The pulsed light is input to the light guide member through the light input end and output through the light output end to be emitted to the wavelength converter. The wavelength-converted light has a fluorescence spectrum having a maximum intensity value in a wavelength range exceeding 710 nm.

An electronic apparatus according to a second aspect of the present invention includes the light emitting device according to the first aspect.

An inspection method according to a third aspect of the present invention uses the light emitting device according to the first aspect.

### BRIEF DESCRIPTION OF DRAWINGS

[Fig. 1] Fig. 1 is a schematic diagram illustrating an example of a light emitting device according to a present embodiment.
[Fig. 2] Fig. 2 is a schematic diagram illustrating another example of the light emitting device according to the present embodiment.
[Fig. 3] Fig. 3 is a schematic diagram illustrating another example of the light emitting device according to the present embodiment.
[Fig. 4] Fig. 4 is a schematic diagram illustrating a configuration of an endoscope according to the present embodiment.
[Fig. 5] Fig. 5 is a schematic diagram illustrating a configuration of an endoscope system according to the present embodiment.
[Fig. 6] Fig. 6 is a graph illustrating an excitation spectrum and a fluorescence spectrum of a phosphor used in example 1.
[Fig. 7] Fig. 7 is a graph illustrating a fluorescence spectrum of a wavelength converter of example 1.
[Fig. 8] Fig. 8 is a graph illustrating fluorescence spectra when the wavelength converter of example 1 is excited by pulsed laser beam of different excitation light densities.
[Fig. 9] Fig. 9 is a graph illustrating fluorescence spectra when the wavelength converter of example 1 is excited by continuous laser beam of different excitation light densities.
[Fig. 10] Fig. 10 is a graph illustrating fluorescence spectra of wavelength converters of examples 2 and 3.

### DESCRIPTION OF EMBODIMENTS

Referring to the drawings, a description is given below of a light emitting device according to a present embodiment, and an electronic apparatus and an inspection method using the light emitting device. Note that dimensional ratios in the drawings are exaggerated for convenience of explanation and are sometimes different from actual ratios.

### [Light emitting device]

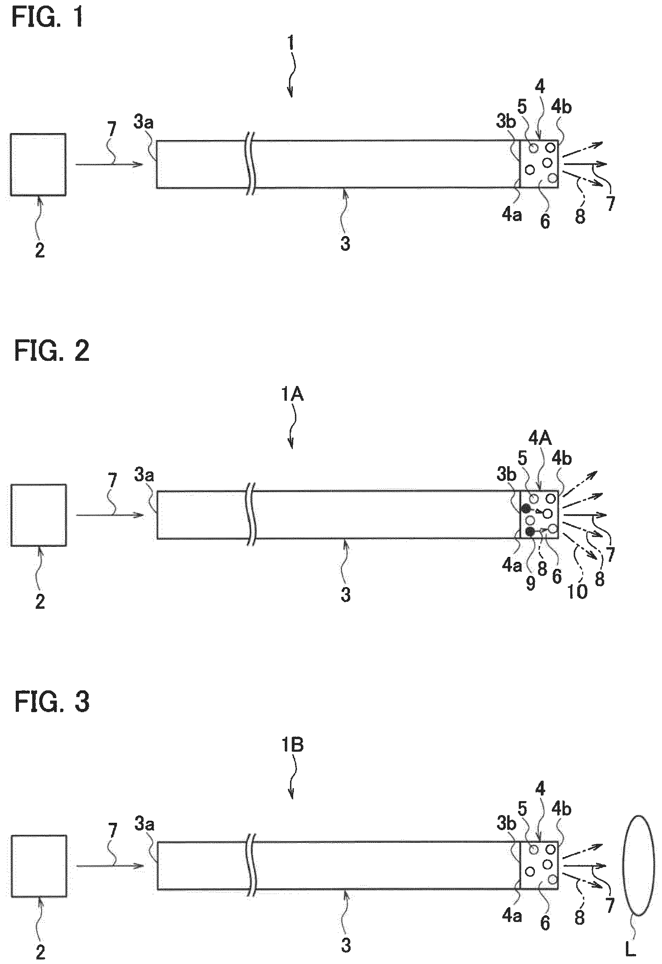

The light emitting device according to the present embodiment is usable in a medical device, for example. As illustrated in Figs. 1 and 2, a light emitting device 1, 1A at least includes an excitation light source 2 provided with a solid-state light emitting element, a light guide member 3 including a light input end 3a and a light output end 3b, and a wavelength converter 4, 4A. The excitation light source 2 is disposed on a light input end 3a side of the light guide member 3, and the wavelength converter 4, 4A is disposed on a light output end 3b side of the light guide member 3. The wavelength converter 4, 4A includes a first phosphor 5 that absorbs a primary light 7 emitted by the excitation light source 2 to emit a wavelength-converted light having a wavelength longer than that of the primary light 7. The light emitting device 1, 1A emits fluorescence from the wavelength converter 4, 4A after the primary light 7 emitted by the excitation light source 2 is guided through the light guide member 3 to be input to the wavelength converter 4, 4A.

### (Excitation light source)

The excitation light source 2 is a light source for emitting the primary light 7 that is a pulsed light. The configuration of the excitation light source 2 is not limited as long as it emits a pulsed light, and a solid-state light emitting element and a function generator are provided, for example. The solid-state light emitting element generates the primary light 7. The function generator is a device that is electrically connected to the solid-state light emitting element and makes the primary light 7 emitted by the solid-state light emitting element into a light (pulsed light) having an arbitrary waveform and a frequency by adjusting a voltage applied to the solid-state light emitting element.

As the solid-state light emitting element, for example, a laser element, such as a surface-emitting laser diode, is used. The output energy of a laser beam emitted by one laser element is preferably 0.1 W or more, more preferably 1 W or more, still more preferably 5 W or more. The energy density of a laser beam emitted by the solid-state light emitting element is preferably 0.5 W/mm² or more, more preferably 2 W/mm² or more, still more preferably 10 W/mm² or more. As is described later, a phosphor in the wavelength converter 4, 4A wavelength-converts the high output laser beam with high efficiency and is hardly deteriorated by the high output laser beam. Thus, the energy density of the laser beam emitted by the solid-state light emitting element is 0.5 W/mm² or more, which enables the light emitting device to emit a high output near-infrared light.

The solid-state light emitting element may be a light emitting diode (LED). For example, using an LED that emits a light with energy of 50 mW or more as the solid-state light emitting element enables the phosphor in the wavelength converter 4, 4A to be excited by high output light. This enables the light emitting device 1, 1A to emit a high output near-infrared light and to exhibit the same effect as that of using a laser element.

As described above, preferably, the excitation light source 2 includes a solid-state light emitting element that is at least one of a laser element or a light emitting diode. However, the solid-state light emitting element is not limited thereto, and any light emitting element is usable as long as it can emit light that can be converted into pulsed light.

Preferably, the excitation light source 2 is a light source that emits the primary light 7, which is a pulsed light having a frequency of 10 Hz or more (100 ms or less) and a duty ratio of 50% or less. With such a configuration, the phosphor is not continuously irradiated with the primary light 7 as the excitation light. Thereby, the phosphor radiates heat while the phosphor is not irradiated with the laser beam, which prevents temperature rise of the phosphor and heat generation of the wavelength converter 4, 4A provided at the light output end 3b of the light guide member 3. It is thus possible to obtain a light emitting device suitable for endoscopic applications, and the like, which hardly causes thermal damage to a living body, as described below. Note that the duty ratio is a ratio of the duration of on/off of pulses in a pulse train that continues at a certain fixed period. In a pulse train of a period T, when the duration in which the pulse is turned on, that is, the duration in which the primary light 7 is emitted by the excitation light source 2 is τ, the duty ratio D is expressed by τ/T.

More preferably, the excitation light source 2 is a light source that emits the primary light 7, which is a pulsed light having a frequency of 50 Hz or more (20 ms or less) and a duty ratio of 50% or less. With such a configuration, the time for irradiating the phosphor with the primary light 7 as the excitation light is shortened. Thus, the phosphor is more easily radiated, which prevents heat generation of the wavelength converter 4, 4A provided at the light output end 3b of the light guide member 3.

Still more preferably, the excitation light source 2 is a light source that emits the primary light 7, which is a pulsed light having a frequency of 100 Hz or more (10 ms or less) and a duty ratio of 50% or less. With such a configuration, the time for irradiating the phosphor with the primary light 7 as the excitation light is further shortened. Thus, the phosphor is further easily radiated, which prevents heat generation of the wavelength converter 4, 4A provided at the light output end 3b of the light guide member 3.

As described above, the duty ratio of the primary light 7 as the pulsed light is preferably 50% or less, more preferably 20% or less, still more preferably 10% or less. This enables the phosphor to further promote heat radiation, which prevents heat generation of the wavelength converter 4, 4A.

Preferably, the excitation light source 2 is a light source that emits the primary light 7, which has an energy density of 0.1 W/mm² or more. In this case, the phosphor in the wavelength converter 4, 4A is excited by the high output primary light 7, which enables the light emitting device 1, 1A to emit a high output near-infrared light. Note that the energy density of the primary light 7 is the energy density while the phosphor in the wavelength converter 4, 4A is irradiated with the primary light 7.

The energy density of the primary light 7 emitted by the excitation light source 2 is more preferably 0.5 W/mm² or more, still more preferably 1 W/mm² or more, particularly preferably 5 W/mm² or more. The upper limit of the energy density of the primary light emitted by the excitation light source 2 is not limited but may be, for example, 50 W/mm².

Preferably, the excitation light source 2 emits the primary light 7, which has a maximum intensity value within a wavelength range of 430 nm to 480 nm. This enables a solid-state light emitting element having high light emission efficiency and emitting blue light to be used to excite the phosphor in the wavelength converter 4, 4A with high efficiency, thereby providing a highly efficient near-infrared light emitting device.

The excitation light source 2 may emit the primary light 7, which has a maximum intensity value within a wavelength range of 250 nm to 420 nm. This enables a solid-state light emitting element having high light emission efficiency and emitting ultraviolet to violet light to be used to excite the phosphor in the wavelength converter 4, 4A with high efficiency, thereby providing a highly efficient near-infrared light emitting device.

The excitation light source 2 may emit the primary light 7, which has a maximum intensity value within a wavelength range of 500 nm to 560 nm. This enables green light of relatively low energy to be used to excite the phosphor in the wavelength converter 4, 4A, thereby providing a highly efficient near-infrared light emitting device suitable for an endoscopic application with less heat generation due to stokes loss of the phosphor.

The excitation light source 2 may emit the primary light 7, which has a maximum intensity value within a wavelength range of 600 nm to 700 nm. This enables red light of relatively low energy to be used to excite the phosphor in the wavelength converter 4, 4A, thereby providing a highly efficient near-infrared light emitting device suitable for an endoscopic application with even less heat generation due to stokes loss of the phosphor.

Thus, preferably, the pulsed light emitted by the excitation light source 2 is ultraviolet light or visible light. This makes it possible to use a phosphor activated with Ce³⁺ or Eu²⁺, which is easy to adjust a fluorescence wavelength by changing a matrix composition. This also makes it possible to excite a phosphor activated by Cr³⁺ haying a strong absorption band in ultraviolet light and visible light regions with high efficiency. This enables the light emitting device 1, 1A to emit a high output near-infrared light.

As described above, the type of the excitation light source 2 provided in the light emitting device 1, 1A is not limited. However, the excitation light source 2 includes preferably three or less types of solid-state light emitting elements, more preferably two or less types, still more preferably one type. With such a configuration, only light of a single wavelength is guided through the light guide member 3, and light of other wavelengths having different light loss properties is not guided through the light guide member. Thus, the light emitting devices 1, 1A is easily designed to prevent light loss. Such a configuration achieves a simple configuration with fewer types of solid-state light emitting elements, thus providing the compact light emitting device 1, 1A.

The light emitting device 1, 1A may further include a laser element that does not excite the wavelength converter 4, 4A. That is, the light emitting device 1, 1A may further include a laser element that emits a laser beam that is not emitted to the wavelength converter 4, 4A. Thus, a light emitting device for a medical device is obtained that uses in combination a medical method using a near-infrared light emitted by the wavelength converter 4, 4A and another treatment using a laser beam emitted by a laser element that does not excite the wavelength converter 4, 4A. In this case, the laser beam emitted by the laser element that does not excite the wavelength converter 4, 4A may be guided by another light guide member different from the light guide member 3 that guides the light exciting the wavelength converter 4, 4A. The laser beam emitted by the laser element that does not excite the wavelength converter 4, 4A may not be guided through the other light guide member. Note that examples of other treatments using a laser beam emitted by a laser element include a treatment using a narrow band light method (narrow band imaging (NBI)), and a laser scalpel treatment.

Preferably, the excitation light source 2 provided in the light emitting device 1, 1A includes multiple solid-state light emitting elements of the same type. Such a configuration enables the wavelength converter 4, 4A to be excited by light of a stronger energy, which provides a light emitting device emitting a higher output near-infrared light.

### (Light guide member)

The light guide member 3 has at least the light input end 3a through which the excitation light from the excitation light source 2 is input, and a light output end 3b through which the incident light is output. The light guide member 3 guides the primary light 7 that is a pulsed light. That is, the primary light 7 that is the pulsed light emitted by the excitation light source 2 is input to the light guide member 3 through the light input end 3a, propagates inside and output through the light output end 3b to be emitted to the wavelength converter 4, 4A. Note that a lens (not shown) may be disposed between the excitation light source 2 and the light guide member 3 to facilitate introduction of the primary light 7 emitted by the excitation light source 2 into the light input end 3a.

The light guide member 3 is not limited as long as it has a function of guiding light, and is preferably an optical fiber, for example. The structure and material of the light guide member 3 are not limited. Examples of the structure of the light guide member 3 include a single mode fiber, a multimode fiber, a double-clad fiber, and a photonic crystal fiber. Examples of the material of the light guide member 3 include quartz, multicomponent glass, plastic, fluoride, and chalcogenide.

### (Wavelength converter)

As illustrated in Figs. 1 and 2, the wavelength converter 4, 4A receives the primary light 7 emitted from the light output end 3b of the light guide member 3 and emits fluorescence having a wavelength longer than that of the primary light 7. The wavelength converter 4, 4A in Figs. 1 and 2 has a configuration to receive the primary light 7 at a front 4a and emit fluorescence from a back 4b. Note that the wavelength converter 4, 4A receives the primary light 7 at the front 4a and may use the fluorescence emitted from the same front 4a as a part of the output light.

The wavelength converter 4, 4A includes the first phosphor 5 that receives the primary light 7 and emits a first wavelength-converted light 8, and a fluorescence spectrum of the first wavelength-converted light 8 has a maximum intensity value in a wavelength range exceeding 710 nm. As a result, the near-infrared light emitted by the light emitting device 1, 1A is easily transmitted through the living body, which provides a near-infrared light emitting device suitable for medical lighting applications.

The fluorescence spectrum of the first wavelength-converted light 8 has a maximum intensity value more preferably in a wavelength range exceeding 750 nm, even more preferably in a wavelength range exceeding 770 nm. As a result, the near-infrared light emitted by the light emitting device 1, 1A is more easily transmitted through the living body, which provides a near-infrared light emitting device suitable for medical lighting applications. Note that preferably, the fluorescence spectrum of the first wavelength-converted light 8 has a maximum intensity value in a wavelength range of 1000 nm or less.

Preferably, the fluorescence spectrum of the first wavelength-converted light 8 has a light component over the entire wavelength range of at least 700 nm to 800 nm. This provides a configuration in which a drug used in the fluorescence imaging method or photodynamic therapy efficiently absorbs the light component of the first wavelength-converted light 8. This also provides a configuration compatible with various types of drugs having different light absorption properties.

More preferably, the first wavelength-converted light 8 has a light component over the entire wavelength range of at least 750 nm to 800 nm. Also preferably, the first wavelength-converted light 8 has a light component over the entire wavelength range of 600 nm to 800 nm. This provides a configuration in which a drug used in the fluorescence imaging method or photodynamic therapy efficiently absorbs the light component of the first wavelength-converted light 8. This also provides a configuration compatible with various types of drugs having different light absorption properties.

The energy density of the first wavelength-converted light 8 emitted to the irradiated object is preferably 0.001 W/mm² or more, more preferably 0.01 W/mm² or more, still more preferably 0.1 W/mm² or more. Preferably, the energy of the first wavelength-converted light 8 is 1 W or more. This provides a configuration in which drugs used in the fluorescence imaging method and photodynamic therapy function more efficiently.

The 1/10 afterglow time of the first wavelength-converted light 8 emitted by the first phosphor 5 is preferably 20 ns to 1000 µs, more preferably 20 ns to less than 100 µs. The 1/10 afterglow time of the first wavelength-converted light 8 is preferably 20 ns to less than 2000 ns, particularly preferably 20 ns to less than 100 ns. Thus, even when the light density of the excitation light for exciting the first phosphor 5 is high, the output of the fluorescence emitted by the first phosphor 5 hardly saturates. Thus, the light emitting device 1, 1A capable of emitting a high output near-infrared light is obtained. Note that the 1/10 afterglow time is a time until the light emission intensity of the phosphor attenuates to 1/10 intensity.

Also preferably, the 1/10 afterglow time of the first wavelength-converted light 8 emitted by the first phosphor 5 is 10 µs or more. This provides a near-infrared light emitting device in which the output light behaves like a continuous light by using the afterglow of the first wavelength-converted light 8 even when a pulsed light such that the supply of the excitation light is temporarily interrupted is used as the primary light 7. Thus, the light emitting device 1, 1A has a configuration suitable for analysis using a continuous light.

The first phosphor 5 included in the wavelength converter 4, 4A is not limited as long as the fluorescence spectrum of the first wavelength-converted light 8 has a maximum intensity value in a wavelength range exceeding 710 nm. However, preferably, the first phosphor 5 includes at least one of a Eu²⁺-activated phosphor or a Ce³⁺-activated phosphor. Ce³⁺ and Eu²⁺ take a mechanism of light absorption and emission based on the 4fₙ⇔4fₙ₋₁5d₁ allowed transition. Thus, the wavelength of absorption and emission changes depending on a matrix crystal to which these are activated. Thus, by selecting an appropriate matrix crystal with Ce³⁺ or Eu²⁺ as an emission center, it is possible to obtain a fluorescent component with a fluorescence spectrum having a maximum intensity value in a wavelength range exceeding 710 nm. Note that in the above-described 4fₙ⇔4fₙ₋₁5d₁ allowed transition, Ce³⁺ corresponds to n=1, and Eu²⁺ corresponds to n=7.

Preferably, the first phosphor 5 includes at least a Cr³⁺-activated phosphor. Cr³⁺ takes a mechanism of light absorption and emission based on the d-d transition. Thus, the wavelength of absorption and emission changes depending on a matrix crystal to which Cr³⁺ is activated. Thus, by selecting an appropriate matrix crystal with Cr³⁺ as an emission center, it is possible to obtain a fluorescent component with a fluorescence spectrum having a maximum intensity value in a wavelength range exceeding 710 nm. The Cr³⁺-activated phosphor converts a laser beam having a high energy density into a near-infrared light with high efficiency, and the fluorescence output hardly saturates even when the laser beam is used. Thus, by using the Cr³⁺-activated phosphor, it is possible to obtain the light emitting device 1, 1A that emits a higher output near-infrared light.

The first phosphor 5 is preferably an oxide-based phosphor, more preferably an oxide phosphor. Note that the oxide-based phosphor means a phosphor including oxygen but not nitrogen, and examples thereof include an alkaline earth metal oxide, an alkaline earth metal haloaluminate, and a rare-earth aluminate each having a calcium ferrite structure.

Since the oxide is stable in the atmosphere, even when the oxide phosphor generates heat due to high density photoexcitation by laser beam, it is difficult for phosphor crystals to be altered by oxidation in the atmosphere, as occurs in nitride phosphors. Thus, when all the phosphor in the wavelength converter 4, 4A is an oxide phosphor, the light emitting device that is highly reliable is obtained.

The first phosphor 5 is preferably a nitride-based phosphor, more preferably a nitride phosphor. The first phosphor 5 is preferably an oxynitride-based phosphor, more preferably an oxynitride phosphor. Since the nitride has a strong covalent bonding property and takes various modifications in terms of composition, it is easy to control the fluorescent color and improve the temperature quenching. Since the thermal conductivity is also excellent, it is advantageous for miniaturization of the light emitting device. Thus, when all the phosphor included in the wavelength converter 4, 4A is a nitride-based phosphor, it is easy to control the color tone of the light emitted by the light emitting device and also to design a compact device.

Preferably, the first phosphor 5 has a garnet crystal structure. Also preferably, the first phosphor 5 is an oxide phosphor with a garnet crystal structure. The phosphor with a garnet structure, especially the oxide phosphor, has a polyhedral particle shape close to a sphere and has excellent dispersibility of a phosphor particle group. Thus, when the phosphor included in the wavelength converter 4, 4A has a garnet structure, the wavelength converter excellent in light transmittance is manufactured relatively easily, which enables higher output of the light emitting device. Further, since the phosphor with a garnet crystal structure has practical experience as a phosphor for LEDs, the light emitting device that is highly reliable is obtained when the first phosphor 5 has the garnet crystal structure.

Preferably, the first phosphor 5 is a phosphor having, as a matrix, a compound having, as a main component, at least one selected from the group consisting of a rare-earth silicate, a rare-earth aluminate, a rare-earth galliate, a rare-earth scandiate, a rare-earth aluminosilicate, an alkaline-earth metal aluminosilicate nitride, and a rare-earth silicate nitride. Preferably, the first phosphor 5 is a phosphor having, as a matrix, at least one selected from the group consisting of a rare-earth silicate, a rare-earth aluminate, a rare-earth aluminosilicate, an alkaline-earth metal aluminosilicate nitride, and a rare-earth silicate nitride. Using the above-described first phosphor 5 enables a part of the primary light 7 to be easily converted into a near-infrared light. Thus, a near-infrared light having a large half width of the fluorescence spectrum is obtained.

Specifically, preferably, the first phosphor 5 is a phosphor having, as a matrix, a compound (A), having, as a main component, at least one selected from the group consisting of RE₂MMg(SiO₄)₃, RE₃Al₂(AlO₄)₃, RE₃Ga₂(GaO₄)₃, RE₃Sc₂(GaO₄)₃, RE₃Sc₂(ScO₄)₃, RE₃Mg₂(SiO₄)₂(AlO₄), MRE₂O₄, MAlSiN₃, and RE₃Si₆N₁₁. Preferably, the first phosphor 5 is a phosphor having, as a matrix, at least one selected from the group consisting of RE₂MMg(SiO₄)₃, RE₃Al₂(ALO₄)₃, RE₃Mg₂(SiO₄)₂(AlO₄), MRE₂O₄, MAlSiN₃, and RE₃Si₆N₁₁. Preferably, the first phosphor 5 is a phosphor having, as a matrix, a solid solution having the compound (A) as an end component. Note that M is an alkaline earth metal, and RE is a rare-earth element.

Preferably, the first phosphor 5 is made from ceramics. This enhances the heat radiation of the first phosphor 5, which prevents the output of the first phosphor 5 from decreasing due to temperature quenching and provides the light emitting device that emits a high output near-infrared light. This also enhances the light absorption efficiency of the first phosphor 5. Thus, it is possible to obtain a light emitting device that emits a high output near-infrared light using a phosphor that efficiently absorbs the excitation light and emits a high output near-infrared light. Here, the ceramic means a sintered body in which particles of the phosphor are bonded to each other.

Preferably, the first phosphor 5 is a ceramic molded body. The ceramic molded body refers to a molded body obtained by sintering a phosphor and an inorganic compound other than the phosphor, or a molded body obtained by sintering only a phosphor. Examples of the ceramic molded body include a molded body formed by sintering a phosphor and a raw material of the phosphor. The first phosphor 5 is a ceramic molded body, and thus the heat radiation property is enhanced, which prevents the decrease in output due to temperature quenching and provides a light emitting device emitting a high output near-infrared light.

As illustrated in Fig. 1, preferably, the wavelength converter 4 further includes a sealing material 6 that disperses the first phosphor 5, in addition to the first phosphor 5. Preferably, the wavelength converter 4 has the first phosphor 5 dispersed in the sealing material 6. By dispersing the first phosphor 5 in the sealing material 6, the primary light 7 emitted by the excitation light source 2 is efficiently absorbed and wavelength-converted into a near-infrared light. Further, the wavelength converter 4 is easily formed into a sheet shape or a film shape.

Preferably, the sealing material 6 is at least one of an organic material or an inorganic material, particularly at least one of a transparent (translucent) organic material or a transparent (translucent) inorganic material. Examples of the sealing material of the organic material include a transparent organic material, such as a silicone resin. Examples of the sealing material of the inorganic material include a transparent inorganic material, such as zinc oxide (ZnO) and a low melting point glass. Using zinc oxide having high thermal conductivity as the sealing material 6 further improves the heat radiation of the wavelength converter 4 and prevents the temperature rise of the wavelength converter 4. As a result, the light emitting device is suitably usable even in a temperature limited environment, such as the inside of a living body.

As illustrated in Fig. 1, the wavelength converter 4 includes the first phosphor 5 that emits the first wavelength-converted light 8. As illustrated in Fig. 2, preferably, the wavelength converter further includes a second phosphor 9 that absorbs the primary light 7 emitted by the excitation light source 2 and emits a second wavelength-converted light 10 that is visible light. Including the second phosphor 9 enables the wavelength converter 4A to emit a white output light by additive color mixing of the primary light 7 emitted by the excitation light source 2, for example a blue laser beam, and the second wavelength-converted light 10.

The second phosphor 9 included in the wavelength converter 4A is not limited as long as it absorbs the primary light 7 emitted by the excitation light source 2 and emits the second wavelength-converted light 10 that is visible light. Preferably, the second phosphor 9 is a Ce³⁺-activated phosphor having, as a matrix, a compound having, as a main component, at least one selected from the compound group consisting of a garnet type crystal structure, a calcium ferrite type crystal structure, and a lanthanum silicon nitride (La₃Si₆N₁₁) type crystal structure. Preferably, the second phosphor 9 is a Ce³⁺-activated phosphor having, as a matrix, at least one component selected from the compound group consisting of a garnet type crystal structure, a calcium ferrite type crystal structure, and a lanthanum silicon nitride type crystal structure. Using the above-described second phosphor 9 provides output light with a large amount of a light component from green to yellow.

Specifically, preferably, the second phosphor 9 is a Ce³⁺-activated phosphor having, as a matrix, a compound (B) having, as a main component, at least one selected from the group consisting of M₃RE₂(SiO₄)₃, RE₃Al₂(AlO₄)₃, MRE₂O₄, and RE₃Si₆N₁₁. Preferably, the second phosphor 9 is a Ce³⁺-activated phosphor having, as a matrix, at least one selected from the group consisting of M₃RE₂(SiO₄)₃, RE₃Al₂(AlO₄)₃, MRE₂O₄, and RE₃Si₆N₁₁. Preferably, the second phosphor 9 is a Ce³⁺-activated phosphor having, as a matrix, a solid solution having the compound (B) as an end component. M is an alkaline-earth metal, and RE is a rare-earth element.

The above-described second phosphor 9 well absorbs a light in a wavelength range of 430 nm to 480 nm and converts it to a green to yellow light having a maximum intensity value in a wavelength range of 540 nm to 590 nm with high efficiency. Thus, a visible light component is easily obtained by using such a phosphor as the second phosphor 9.

When the wavelength converter 4A includes the first phosphor 5 and the second phosphor 9, preferably, the first phosphor 5 emits the first wavelength-converted light 8 by absorbing at least one of the primary light 7 emitted by the excitation light source 2 or the second wavelength-converted light 10 emitted by the second phosphor 9. As described above, preferably, the first phosphor 5 is a phosphor that absorbs the primary light 7 emitted by the excitation light source 2 and emits the first wavelength-converted light 8 that is near-infrared light. The first phosphor 5 may be a phosphor that absorbs the second wavelength-converted light 10 emitted by the second phosphor 9 and emits the first wavelength-converted light 8 that is near-infrared light. That is, the second phosphor 9 may be excited by the primary light 7 to emit the second wavelength-converted light 10, and the first phosphor 5 may be excited by the second wavelength-converted light 10 to emit the first wavelength-converted light 8. In this case, even if the first phosphor 5 is a phosphor that is hardly excited by the primary light 7, by interposing the second phosphor 9, the first phosphor 5 is excite by the fluorescence emitted by the second phosphor 9. As a result, a phosphor that absorbs visible light is selectable as the first phosphor 5, which expands the choice of the first phosphor 5 and facilitates the industrial production of the light emitting device. When the first phosphor 5 absorbs the second wavelength-converted light 10 and emits the first wavelength-converted light 8, the light emitting device is capable of emitting the first wavelength-converted light 8 having a large near-infrared light component intensity.

Preferably, the wavelength converter 4, 4A is made from an inorganic material. Here, the inorganic material means materials other than organic materials and includes ceramics and metals as a concept. When the wavelength converter 4, 4A is made from an inorganic material, the wavelength converter 4, 4A has the thermal conductivity higher than that of the wavelength converter including an organic material, such as a sealing resin, thereby facilitating the heat radiation design. Thus, the temperature rise of the wavelength converter 4, 4A is effectively prevented even when the phosphor is photoexcited with high density by the primary light 7 emitted by the excitation light source 2. As a result, the temperature quenching of the phosphor in the wavelength converter 4, 4A is prevented, and thus higher output of light emission is possible.

As described above, the wavelength converter 4, 4A is preferably made from an inorganic material, and thus the sealing material 6 is preferably made from an inorganic material. Preferably, zinc oxide (ZnO) is used as the inorganic material. This further enhances the heat radiation of the phosphor, which prevents the output of the phosphor from decreasing due to temperature quenching and provides the light emitting device that emits a high output near-infrared light.

Note that the wavelength converter 4, 4A may be a wavelength converter that does not use the sealing material 6. In this case, the wavelength converter 4, 4A may be made of only a phosphor. The wavelength converter 4, 4A may be formed by fixing particles of a phosphor to each other using an organic or inorganic binder. As the binder, a commonly used resin-based adhesive, ceramic fine particles, low melting point glass, or the like is usable. The wavelength converter not using the sealing material 6 is made thin, and thus it is suitably used for the light emitting device.

Next, the operation of the light emitting device according to the present embodiment is described. In the light emitting device 1 in Fig. 1, first, the primary light 7 emitted by the excitation light source 2 is emitted to the light input end 3a of the light guide member 3. In this case, a lens may be disposed between the excitation light source 2 and the light guide member 3 to condense the primary light 7 and to facilitate introduction of the primary light 7 to the light input end 3a. The primary light 7 input to the light guide member 3 through the light input end 3a propagates inside the light guide member 3 and is output through the light output end 3b. The primary light 7 output through the light output end 3b is emitted to the front 4a of the wavelength converter 4. The emitted primary light 7 passes through the wavelength converter 4. When the primary light 7 passes through the wavelength converter 4, the first phosphor 5 included in the wavelength converter 4 absorbs a part of the primary light 7 and emits the first wavelength-converted light 8. In this way, a light including the primary light 7 and the first wavelength-converted light 8 is emitted from the back 4b of the wavelength converter 4 as output light.

In the light emitting device 1A in Fig. 2, the primary light 7 output through the light output end 3b is emitted to the front 4a of the wavelength converter 4A. The emitted primary light 7 passes through the wavelength converter 4A. When the primary light 7 passes through the wavelength converter 4A, the second phosphor 9 included in the wavelength converter 4A absorbs a part of the primary light 7 and emits the second wavelength-converted light 10. Further, the first phosphor 5 included in the wavelength converter 4A absorbs a part of the primary light 7 and/or a part of the second wavelength-converted light 10 to emit the first wavelength-converted light 8. Thus, a light including the primary light 7, the first wavelength-converted light 8, and the second wavelength-converted light 10 is emitted from the back 4b of the wavelength converter 4A as output light.

As described above, the light emitting device 1, 1A according to the present embodiment includes a solid-state light emitting element that emits a pulsed light (primary light 7), and the wavelength converter 4, 4A including a phosphor (first phosphor 5) that emits' a wavelength-converted light (first wavelength-converted light 8) having a wavelength longer than that of the pulsed light. The light emitting device 1, 1A further includes the light guide member 3 having the light input end 3a and the light output end 3b. The wavelength converter 4, 4A is disposed on the light output end 3b side of the light guide member 3. The pulsed light is input to the inside of the light guide member 3 through the light input end 3a and output through the light output end 3b to be emitted to the wavelength converter 4, 4A. The fluorescence spectrum of the wavelength-converted light has a maximum intensity value in a wavelength range exceeding 710 nm.

In the light emitting device 1, 1A, since the wavelength converter 4, 4A is irradiated with the pulsed light, the phosphor radiates heat while not irradiated with the excitation light, and the temperature rise of the phosphor is prevented. Thus, even when the wavelength converter 4, 4A is disposed on the light output end 3b of the light guide member 3, heat generation at the light output end 3b is prevented. Thus, even when the light output end 3b touches a living body or the like, the light output end 3b is unlikely to cause thermal damage to the living body, which provides a light emitting device suitable for endoscopic applications, for example. As a result, the light emitting device 1 according to the present embodiment is suitably usable even in a temperature limited environment, such as inside a living body. In contrast, when the wavelength converter 4, 4A is continuously irradiated with the laser beam, the temperature of the light output end 3b rises due to the heat generation of the near-infrared light emitting phosphor having large stokes loss, which makes it difficult to be used in a temperature limited environment.

In the light emitting device 1, 1A, the pulsed light (primary light 7) emitted by the excitation light source 2 has a strong directivity, which enables the pulsed light emitted by the excitation light source 2 to be introduced into the light guide member 3 from the light input end 3a with a high coupling efficiency. Thus, the diameter of the light guide member 3 is reduced without difficulty. In contrast, if the wavelength converter 4, 4A is disposed between the excitation light source 2 and the light input end 3a of the light guide member 3, the fluorescence emitted by the wavelength converter 4, 4A is diffused all around the wavelength converter 4, 4A. Thus, it becomes difficult to introduce the fluorescence emitted by the wavelength converter 4, 4A into the light guide member 3 through the light input end 3a, and the efficiency of coupling light to the light guide member 3 is reduced.

Further, in the light emitting device 1, 1A, the pulsed light is input to the light guide member 3 through the light input end 3a and output through the light output end 3b to be emitted to the wavelength converter 4, 4A. That is, the pulsed light is input into the light guide member 3, but fluorescence emitted by the wavelength converter 4, 4A is not input into the light guide member 3. Thus, only the pulsed light is guided through the light guide member 3, and the fluorescent component whose wavelength has been converted to a long wavelength is not guided through the light guide member at all. In general, a light guide member, such as an optical fiber, has a property of absorbing near-infrared light. Thus, when the wavelength converter 4, 4A is arranged between the excitation light source 2 and the light input end 3a of the light guide member 3, the fluorescence emitted by the wavelength converter 4, 4A is absorbed by the light guide member 3, and the light loss tends to increase. In contrast, in the light emitting device 1, 1A, only the pulsed light having a relatively small absorption loss by the light guide member 3 is guided through the light guide member 3, and the fluorescent component whose wavelength has been converted to a long wavelength is not guided through the light guide member. This reduces the light loss by the light guide member 3 and enhances the light emission efficiency of the light emitting devices 1, 1A.

In this way, the light emitting device 1, 1A irradiates the wavelength converter 4,4A disposed at the light output end 3b while the excitation light (pulsed light) maintains a large intensity. The wavelength converter 4, 4A converts the excitation light into near-infrared light having substantially no directivity and emits it. Thus, the near-infrared light having a high output power close to that of a point light source and having a Lambertian light distribution is output from the tip of the thin light guide member 3, which makes the light emitting device highly efficient and suitable for endoscope lighting.

In Figs. 1 and 2, the wavelength converter 4, 4A is in contact with the light output end 3b of the light guide member 3, but the light emitting device 1, 1A is not limited to such a configuration. That is, a gap may exist between the wavelength converter 4, 4A and the light output end 3b of the light guide member 3. Another member, for example, a lens, may be interposed between the wavelength converter 4, 4A and the light output end 3b of the light guide member 3.

The light emitting device 1, 1A according to the present embodiment may further include a lens for condensing the wavelength-converted light on the light output end 3b side of the light guide member 3. Specifically, as illustrated in Fig. 3, a light emitting device IB may further include a lens L that is disposed on a surface (back 4b) side of the wavelength converter 4 (4A), the surface being opposite to a surface (front 4a) facing the light output end 3b of the light guide member 3, and condenses the wavelength-converted light. Such a configuration enables the focused wavelength-converted light of high energy density (high illuminance) to be emitted to a fluorescent drug used in the fluorescence imaging method and a photosensitive substance used in the photodynamic therapy, thereby making the fluorescent drug and the photosensitive substance function more efficiently. This provides a light emitting device suitable for lighting for the fluorescence imaging method and lighting for the photodynamic therapy.

The light emitting device 1, 1A according to the present embodiment may be used for optical coherence tomography (OCT) or the like. Preferably, the light emitting device 1, 1A is a medical light emitting device used in a fluorescence imaging method or a photodynamic therapy. These methods are a promising medical technology with a wide range of applications and are highly practical. The light emitting device 1, 1A illuminates the inside of the living body with a broad near-infrared high output light through the "window of living body" and makes the fluorescent drug or photosensitive drug taken into the living body fully functional, which is expected to have a large therapeutic effect.

The fluorescence imaging method is a method of observing a lesion by administering a fluorescent drug that selectively binds to the lesion, such as a tumor, to a subject, exciting the fluorescent drug with a specific light, and detecting and imaging fluorescence emitted by the fluorescent drug with an image sensor. The fluorescence imaging method makes it possible to observe lesions that are difficult to observe using only general lighting. As the fluorescent drug, a drug that absorbs excitation light in the near-infrared range, and emits fluorescence in the near-infrared range and at a wavelength longer than that of the excitation light is usable. Examples of the fluorescent drug used include at least one selected from the group consisting of indocyanine green (ICG), a phthalocyanine-based compound, a talaporfin sodium-based compound, and a dipicolylcyanine (DIPCY)-based compound.

The photodynamic therapy is a treatment method of administering a photosensitive drug that selectively binds to a target biological tissue to a subject and irradiating the photosensitive drug with near-infrared light When the photosensitive drug is irradiated with the near-infrared light, the photosensitive drug generates reactive oxygen, which is usable to treat lesions, such as tumors or infections. Examples of the photosensitive drug used include at least one selected from the group consisting of a phthalocyanine-based compound, a porphyrin-based compound, a chlorin-based compound, a bacteriochlorin-based compound, a psoralen-based compound, a porfimer sodium-based compound, and a talaporfin sodium-based compound.

The light emitting device 1, 1A according to the present embodiment may be used as a light source for a sensing system or a lighting system for a sensing system. That is, further providing the light emitting device 1, 1A with an orthodox light receiving element having light receiving sensitivity in the near-infrared wavelength range makes it possible to construct a highly-sensitivity sensing system. Such a light emitting device facilitates miniaturization of the sensing system and broadening of the sensing range.

### [Medical device]

Next, a medical device according to the present embodiment is described. Specifically, as an example of the medical device, an endoscope provided with the light emitting device and an endoscope system using the endoscope are described.

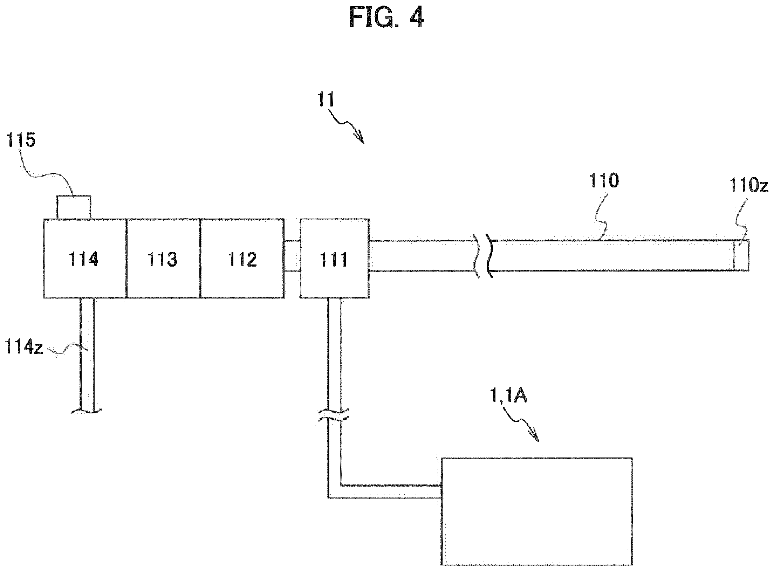

The endoscope according to the present embodiment includes the above-described light emitting device 1, 1A. Specifically, as illustrated in Fig. 4, an endoscope 11 includes a scope 110, a light source connector 111, a mount adapter 112, a relay lens 113, a camera head 114, an operation switch 115, and the light emitting device 1, 1A.

The scope 110 is an elongated tube capable of guiding light to the tip and is inserted into the body when in use. The scope 110 includes an imaging window 110z at its tip. For the imaging window 110z, an optical material, such as optical glass or optical plastic, is used. The scope 110 includes, for example, the light guide member 3 illustrated in Fig. 1 or 2, and the optical fiber.

The light guide member 3 is a member for guiding the primary light 7 emitted by the excitation light source 2, and the optical fiber is used for transmitting an optical image input to the imaging window 110z. Note that the light guide member 3 may be protected by plastic or metal to prevent leakage of light to the outside or breakage.

The primary light 7 emitted by the excitation light source 2 is guided to the tip of the scope 110 through the light guide member 3 and is emitted to the wavelength converter 4, 4A. Then, the first wavelength-converted light 8 subjected to the wavelength conversion by the wavelength converter 4, 4A is emitted to the diseased part or the like in the body.

In contrast, an optical signal from the diseased part obtained by irradiation with the first wavelength-converted light 8 is input to the imaging window 110z, taken into the scope 110, and transmitted to a detector provided inside the endoscope 11.

In the endoscope 11, the imaging window 110z is not essential, and the first wavelength-converted light 8 may be emitted to a diseased part or the like in the body without passing through the imaging window 110z.

The light source connector 111 connects the light guide member 3 and the scope 110. The mount adapter 112 is a member for mounting the scope 110 on the camera head 114. Various scopes 110 are detachably mountable on the mount adapter 112.

The relay lens 113 converges the optical image transmitted through the scope 110 on an imaging surface of an image sensor. The relay lens 113 may be moved in accordance with the operation amount of the operation switch 115 to perform focus adjustment and magnification adjustment.

The camera head 114 includes a color separation prism inside. The color separation prism separates, for example, the light converged by the relay lens 113 into R light (red light), G light (green light), and B light (blue light). Note that the color separation prism may further separate an IR light (near-infrared light). This also makes the endoscope 11 capable of identifying a lesion part using the fluorescence imaging method using near-infrared light.

The camera head 114 further includes image sensors as detectors inside. The image sensors each convert an optical image formed on each imaging surface into an electric signal. The image sensors are not limited, but at least one of CCD (Charge Coupled Device) or CMOS (Complementary Metal Oxide Semiconductor) is usable. The image sensors are dedicated sensors for each receiving B component (blue component), R component (red component), or G component (green component) light, for example. Note that the camera head 114 may further include a dedicated sensor for receiving an IR component (near-infrared component). This also makes the endoscope 11 capable of identifying a lesion part using the fluorescence imaging method using near-infrared light.

The camera head 114 may have a color filter inside instead of the color separation prism. The color filter is provided on the imaging surface of the image sensor. For example, three color filters are provided, and the three color filters receive the light converged by the relay lens 113 and selectively transmit R light (red light), G light (green light), and B light (blue light), respectively. Note that the color filter for selectively transmitting an IR light (near-infrared light) is further provided, which makes the endoscope 11 also capable of specifying a lesion part using a fluorescence imaging method using near-infrared light.

When the fluorescence imaging method using near-infrared light is used, preferably, the color filter for selectively transmitting IR light includes a barrier film for cutting the reflection component of near-infrared light (IR light) included in the lighting. This enables only the fluorescence composed of IR light emitted by a fluorescent drug used in the fluorescence imaging method to form an image on the imaging surface of an image sensor for IR light. Therefore, the diseased part luminous with the fluorescent drug is easily observed clearly.

As illustrated in Fig. 4, a signal cable 114z is connected to the camera head 114 to transmit an electric signal from an image sensor to a CCU 12 described later.

In the endoscope 11 having such a configuration, light from a subject is guided to the relay lens 113 through the scope 110 and is further transmitted through the color separation prism in the camera head 114 to form images on the image sensors.

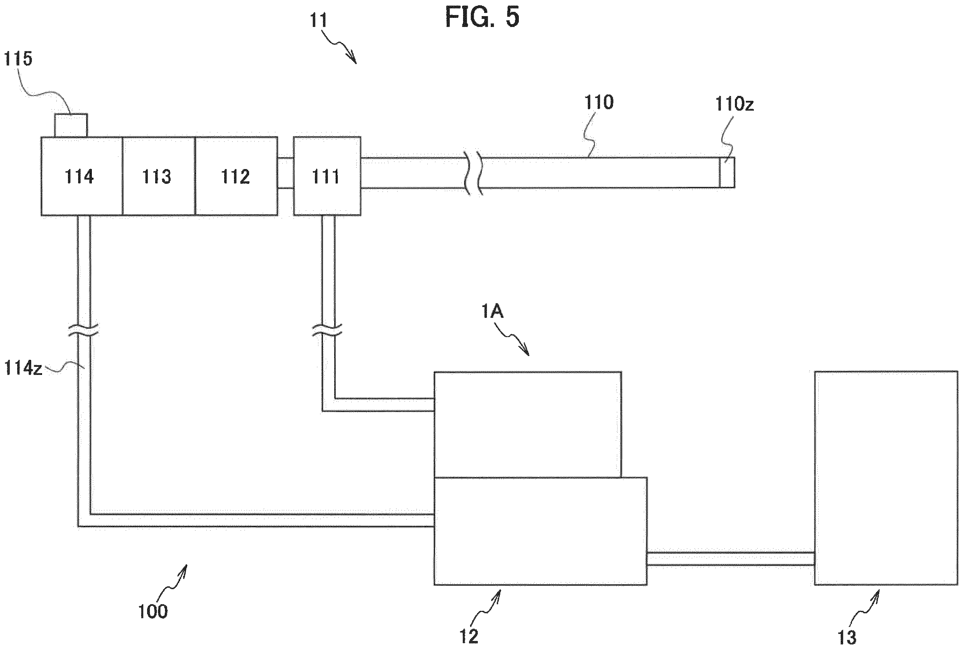

As illustrated in Fig. 5, the endoscope system 100 according to the present embodiment includes the endoscope 11 for imaging the inside of a subject, a CCU (Camera Control Unit) 12, and a display device 13, such as a display. The endoscope 11 includes the light emitting device 1A.

The CCU 12 includes at least an RGB signal processing unit, an IR signal processing unit, and an output unit. The CCU 12 executes a program stored in the internal or external memory of the CCU 12 to implement the respective functions of the RGB signal processing unit, the IR signal processing unit, and the output unit.

The RGB signal processing unit converts the electrical signals of the B component, the R component, and the G component from the image sensors into video signals that are displayable on the display device 13 and outputs the video signals to the output unit. The IR signal processing unit converts the electrical signal of the IR component from the image sensor into a video signal and outputs the video signal to the output unit.

The output unit outputs at least one of the video signals of respective RGB color components or the video signal of the IR component to the display device 13. For example, the output unit outputs video signals on the basis of either a simultaneous output mode or a superimposed output mode.

In the simultaneous output mode, the output unit simultaneously outputs the RGB image and the IR image on separate screens. The simultaneous output mode enables the diseased part to be observed by comparing the RGB image and the IR image on the separate screens. In the superimposed output mode, the output unit outputs a composite image in which the RGB image and the IR image are superimposed. The superimposed output mode enables the diseased part luminous with the fluorescent drug used in the fluorescence imaging method to be clearly observed in the RGB image, for example.

The display device 13 displays an image of a target, such as a diseased part, on a screen on the basis of video signals from the CCU 12. In the simultaneous output mode, the display device 13 divides the screen into multiple screens and displays the RGB image and the IR image side by side on each screen. In the superimposed output mode, the display device 13 displays a composite image in which an RGB image and an IR image are superimposed on each other on a single screen.

As described above, the endoscope 11 according to the present embodiment includes the light emitting device 1, 1A and emits the first wavelength-converted light 8 through the imaging window 110z of the scope 110. Specifically, the endoscope 11 is provided with the light guide member 3 inside the scope 110, and the light output end 3b of the light guide member 3 is disposed near the imaging window 110z of the scope 110. Further, for example, the wavelength converter 4, 4A is disposed between the light output end 3b and the imaging window 110z. Then, the pulsed light emitted by the excitation light source 2 propagates through the light guide member 3 of the scope 110 and is emitted to the wavelength converter 4, 4A from the light output end 3b. Then, the first wavelength-converted light 8 is emitted from the wavelength converter 4, 4A, passes through the imaging window 110z, and is emitted to the diseased part in the body. Thus, by using the endoscope 11, it is possible to efficiently excite a photosensitive substance accumulated in the diseased part of the body and make the photosensitive substance function sufficiently.

When the endoscope 11 is provided with the light emitting device 1A including the second phosphor 9, the endoscope 11 emits the second wavelength-converted light 10 that is visible light in addition to the first wavelength-converted light 8. Thus, by using the endoscope system 100, it is possible to irradiate the diseased part with the first wavelength-converted light 8 while specifying the position of the diseased part.

As described above, the medical device according to the present embodiment includes the light emitting device 1, 1A. Since the light emitting device 1, 1A prevents heat generation of the wavelength converter 4, 4A disposed at the light output end 3b of the light guide member 3, the vicinity of the light output end 3b is hardly heated. Thus, for example, even when the tip of the scope 110 of the endoscope 11 touches a living body or the like, the living body is hardly thermally damaged. Thus, the medical device according to the present embodiment is suitably usable even in a temperature limited environment, such as inside a living body.

Preferably, the endoscope 11 according to the present embodiment further includes a detector for detecting fluorescence emitted by a fluorescent drug that has absorbed the first wavelength-converted light 8. By providing the endoscope 11 with the detector for detecting fluorescence emitted by a fluorescent drug in addition to the light emitting device 1, the diseased part is specified only by the endoscope. This makes it possible to perform medical examination and treatment with less burden on the patient, since there is no need to open the abdomen wide to identify the diseased part as in the conventional method. This also enables the doctor using the endoscope 11 to accurately identify the diseased part, which improves the efficiency of treatment.

Preferably, the medical device according to the present embodiment is used for either the fluorescence imaging method or photodynamic therapy. This medical device illuminates the inside of the living body with a broad near-infrared high output light and makes the fluorescent drug or photosensitive drug taken into the living body fully functional, which is expected to have a large therapeutic effect. Such a medical device uses the light emitting device 1, 1A having a relatively simple configuration, which is advantageous in reducing the size and the cost.

### [Electronic apparatus]

Next, an electronic apparatus according to the present embodiment is described. The electronic apparatus according to the present embodiment includes the light emitting device 1, 1A. As described above, the light emitting device 1, 1A is expected to have a large therapeutic effect, and it is easy to miniaturize the sensing system. Since the electronic apparatus of the present embodiment is provided with the light emitting device 1, 1A, when this electronic apparatus is used for a medical device or a sensing device, a large therapeutic effect, miniaturization of the sensing system, and the like are expected.

The electronic apparatus includes, for example, the light emitting device 1, 1A and a light receiving element. The light receiving element is, for example, a sensor, such as an infrared sensor for detecting light in a near-infrared wavelength range. Preferably, the electronic apparatus is any of an information recognition device, a sorting device, a detection device, or an inspection device. As described above, these devices also facilitate miniaturization of the sensing system and broadening of the sensing range.

The information recognition device is, for example, a driver support system that recognizes the surrounding situation by detecting reflected components of emitted infrared rays.

The sorting device is, for example, a device that sorts an irradiated object into predetermined categories by using the difference in infrared light components between the irradiation light and reflected light reflected by the irradiated object.

The detection device is, for example, a device that detects a liquid. Examples of liquids include water, and flammable liquids that are prohibited from being transported in aircraft. Specifically, the detection device may be a device for detecting moisture adhering to glass, and moisture absorbed by an object, such as sponge or fine powder. The detection device may visualize the detected liquid. Specifically, the detection device may visualize the distribution information of the detected liquid.

The inspection device may be any of a medical inspection device, an agricultural and livestock industry inspection device, a fishery inspection device, or an industrial inspection device. These devices are useful for inspecting an inspection target in each industry.

The medical inspection device is, for example, an examination device that examines the health condition of a human or non-human animal. Non-human animals are, for example, domestic animals. The medical inspection device is, for example, a device used for a biological examination, such as a fundus examination or a blood oxygen saturation examination, and a device used for examination of an organ, such as a blood vessel or an organ. The medical inspection device may be a device for examining the inside of a living body or a device for examining the outside of a living body.

The agricultural and livestock industry inspection device is, for example, a device for inspecting agricultural and livestock products including agricultural products and livestock products. Agricultural products may be used as foods, for example, fruits and vegetables, or cereals, or as fuels, such as oils. Livestock products include, for example, meat and dairy products. The agricultural and livestock industry inspection device may be a device for non-destructively inspecting the inside or outside of the agricultural and livestock products. Examples of the agricultural and livestock industry inspection device includes a device for inspecting the sugar content of vegetables and fruits, a device for inspecting the acidity of vegetables and fruits, a device for inspecting the freshness of vegetables and fruits by the visualization of leaf veins, a device for inspecting the quality of vegetables and fruits by the visualization of wounds and internal defects, a device for inspecting the quality of meat, and a device for inspecting the quality of processed foods processed with milk, meat, or the like as raw materials.

The fishery inspection device is, for example, a device for inspecting the flesh quality of fish, such as tuna, or a device for inspecting the presence or absence of the contents in shells of shellfish.

The industrial inspection device is, for example, a foreign matter inspection device, a content inspection device, a condition inspection device, or a structure inspection device.

Examples of the foreign matter inspection device include a device for inspecting foreign matter in a liquid contained in a container, such as a beverage or a liquid medicine, a device for inspecting foreign matter in a packaging material, a device for inspecting foreign matter in a printed image, a device for inspecting foreign matter in a semiconductor or an electronic component, a device for inspecting foreign matter, such as residual bone in food, dust, or machine oil, a device for inspecting foreign matter in processed food in a container, and a device for inspecting foreign matter in medical devices, such as adhesive plasters, medical and pharmaceutical products, or quasi-drugs.

Examples of the content inspection device include a device for inspecting the content of a liquid contained in a container, such as a beverage or a liquid medicine, a device for inspecting the content of a processed food contained in a container, and a device for inspecting the content of asbestos in building materials.

Examples of the condition inspection device include a device for inspecting packaging state of a packaging material, and a device for inspecting printing state of a packaging material.

Examples of the structure inspection device include an internal non-destructive inspection device and an external non-destructive inspection device for a composite member or a composite component, such as a resin product. A specific example of the resin product is, for example, a metal brush with a part of metal wire embedded in the resin, and the inspection device inspects the bonding state of the resin and the metal.

The electronic apparatus according to the present embodiment may use color night vision technology. The color night vision technology uses a correlation of reflection intensity between visible light and infrared rays to colorize an image by assigning infrared rays to RGB signals for each wavelength. According to the color night vision technology, a color image is obtained only by infrared rays, and it is particularly suitable for a security device.

As described above, the electronic apparatus includes the light emitting device 1, 1A. When the light emitting device 1, 1A includes a power source, the excitation light source 2, and the wavelength converter 4, 4A, it is not necessary to accommodate all of them in one housing. Thus, the electronic apparatus according to the present embodiment provides a highly accurate and compact inspection method, or the like, with excellent operability.

### [Inspection method]

Next, an inspection method according to the present embodiment is described. As described above, the electronic apparatus including the light emitting device 1, 1A is also usable as an inspection device. That is, the light emitting device 1, 1A is usable in the inspection method according to the present embodiment. This provides a highly accurate and compact inspection method with excellent operability.

### EXAMPLES

The light emitting device according to the present embodiment is described below in more detail with reference to examples, but the present embodiment is not limited thereto.

### [Preparation of phosphor]

Oxide phosphors used in examples were synthesized using a preparation method utilizing a solid phase reaction. In the present examples, the following compound powders were used as raw materials.
Gadolinium oxide (Gd₂O3): purity 3N, Wako Pure Chemical Corporation
Lanthanum oxide (La₂O₃): purity 3N, Wako Pure Chemical Corporation
Scandium oxide (Sc₂O₃): Purity 3N, Shin-Etsu Chemical Co., Ltd.
Gallium oxide (Ga₂O3): purity 4N, Wako Pure Chemical Corporation
Chromium oxide (Cr₂O₃): purity 3N, Kojundo Chemical Laboratory Co., Ltd.

### (Example 1)

The phosphor used in example 1 is an oxide phosphor represented by a formula: Gd₃(Ga_{0.97}Cr_{0.03})₂Ga₃O₁₂. The phosphor of example 1 is a Cr³⁺-activated phosphor.

First, the above-described raw materials were weighed to obtain a compound of a stoichiometric composition Gd₃(Ga_{0.97}Cr_{0.03})₂Ga₃O₁₂. The weighed raw materials were then put into a beaker containing pure water and stirred with a magnetic stirrer for 1 hour. Thus, a slurry-like mixed raw material of the pure water and raw materials was obtained. Then, the slurry-like mixed raw material was dried entirely using a dryer. The mixed raw material after drying was pulverized using a mortar and a pestle to obtain a raw material for calcining.

The above-described raw material for calcining was transferred to a small alumina crucible and calcined in air at 1450 °C for 1 hour in a box-type electric furnace to obtain the phosphor used in example 1. Note that the temperature rise and fall rate inside the electric furnace was set at 400 °C/h. The phosphor used in example 1 is a powdery phosphor. The body color of the obtained phosphor was dark green.

### (Example 2)

The phosphor used in example 2 is an oxide phosphor represented by a formula: (Gd_{0.80}La_{0.20})₃(Sc_{0.50}Ga_{0.47}Cr_{0.03})₂Ga₃O₁₂. The phosphor of example 2 is also a Cr³⁺-activated phosphor.

First, the above-described raw materials were weighed to obtain a compound of a stoichiometric composition (Gd_{0.80}La_{0.20})₃(Sc_{0.50}Ga_{0.47}Cr_{0.03})₂Ga₃O₁₂. Next, the weighed raw materials were put into a plastic pot, pure water and an alumina ball were added, and mixed using a ball mill for several hours. Thus, a slurry of the pure water and raw materials was obtained. Then, the slurry was dried entirely using a dryer to obtain a mixed raw material. Then, after the mixed raw material was lightly mixed using a mortar and pestle, the mixed raw material was put into a metal mold (inner diameter 10 mm, height 30 mm) and pressurized at a pressure of 250 atm. In this way, a molded body of the raw material of example 2 was obtained.

The obtained molded body was calcined in air at 1500 °C for 2 hours using a box-type electric furnace to obtain a phosphor as a ceramic compact used in example 2. Note that the temperature rise and fall rate inside the electric furnace was set at 400 °C /h. The body color of the obtained phosphor was dark green.

### (Example 3)

The phosphor used in example 3 is an oxide phosphor represented by a formula: (Gd_{0.75}La_{0.25})₃(Sc_{0.50}Ga_{0.47}Cr_{0.03})₂Ga₃O₁₂. The phosphor of example 3 is also a Cr³⁺-activated phosphor.

First, the raw material was weighed so as to obtain a compound having a stoichiometric composition (Gd_{0.75}La_{0.25})₃(Sc_{0.50}Ga_{0.47}Cr_{0.03})₂Ga₃O₁₂. Next, as in example 2, a mixed raw material was obtained by mixture and drying, which was then pressurized to obtain a molded body of the raw material of example 3.

The obtained molded body was calcined in air at 1500 °C for 2 hours in the same manner as in example 2 to obtain a phosphor as a ceramic compact used in example 3. Note that the body color of the obtained phosphor was dark green.

### [Evaluation]

### (Crystal structure analysis)

Next, the crystal structures of the phosphors used in examples 1 to 3 were evaluated using an X-ray diffraction apparatus (MiniFlex manufactured by Rigaku Corporation).

As a result of evaluation, it was found that the phosphors used in examples 1 to 3 were all made from compounds with a garnet crystal structure, although the details are omitted. That is, the phosphors used in examples 1 to 3 were found to be garnet phosphors. In this way, it was confirmed that the phosphors used in examples 1, 2, and 3 were Gd₃(Ga_{0.97}Cr_{0.03})₂Ga₃O₁₂, (Gd_{0.80}La_{0.20})₃(Sc_{0.50}Ga_{0.47}Cr_{0.03})₂Ga₃O₁₂, and (Gd_{0.75}La _{0.25})₃(Sc_{0.50}Ga_{0.47}Cr_{0.03})₂Ga₃O₁₂ as compounds, respectively.

### (Spectroscopic property)

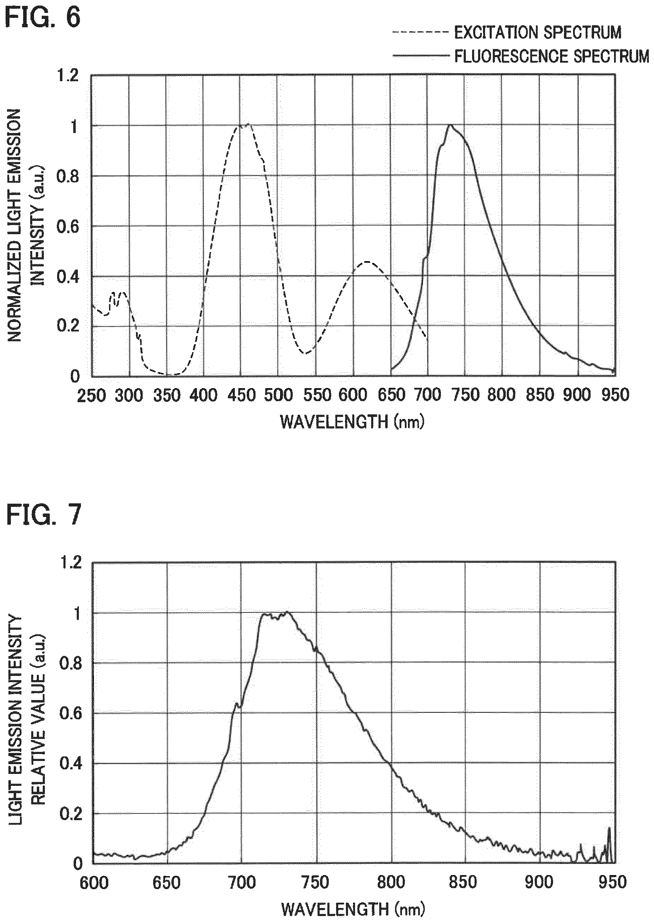

Next, the excitation property and the fluorescence property of the phosphor used in example 1 are evaluated using a spectrofluorometer (FP-6500 manufactured by JASCO Corporation). Fig. 6 illustrates an excitation spectrum and a fluorescence spectrum of the phosphor used in example 1. Note that an excitation wavelength at the time of the fluorescence spectrum measurement was set to 450 nm, and a monitor wavelength at the time of the excitation spectrum measurement was set to a fluorescence peak wavelength. In Fig. 6, the fluorescence spectrum and the excitation spectrum are normalized to have a maximum intensity value of 1.

As is seen from Fig. 6, the fluorescence spectrum of the phosphor used in example 1 was a broad spectrum determined to be attributed to the d-d transition of Cr³⁺. As is seen from Fig. 6, the phosphor used in example 1 has strong excitation bands in a wavelength range of 250 nm to 310 nm, a wavelength range of 400 nm to 500 nm, and a wavelength range of 580 nm to 680 nm. That is, the phosphor used in example 1 was a phosphor that strongly absorbed ultraviolet light, violet to blue-green light, and orange to deep-red light and emitted fluorescence.

Although the details are omitted, the phosphors used in example 2 and example 3 were evaluated in the same manner as described above, and as a result, all phosphors had strong excitation bands in three wavelength ranges. The excitation bands of the phosphor are located in a wavelength range of 250 nm to 310 nm, a wavelength range of 400 nm to 500 nm, and a wavelength range of 580 nm to 680 nm, respectively. Thus, the phosphors used in example 2 and example 3 were phosphors that strongly absorbed ultraviolet light, violet to blue-green light, and orange to deep-red light and emitted fluorescence having a fluorescence peak at around 750 nm.

The above shows that the phosphors used in example 1 to 3 are excited by a solid-state light emitting element that emits at least one type of light selected from ultraviolet, violet, blue, blue-green, orange, red, or deep-red. It is shown that the phosphors used in example 1 to 3 wavelength-convert the excitation light into fluorescence that forms a band spectrum that includes a large amount of a light component in the near-infrared light range.

### (Afterglow time)

Using a compact fluorescence lifetime measurement device Quantaurus-Tau (registered trademark) (C11367 manufactured by Hamamatsu Photonics K.K.), the 1/10 afterglow time of the phosphor used in example 1 was measured. As a result of evaluation, the 1/10 afterglow time of the phosphor used in example 1 was 383 µs.

The phosphors used in examples 2 and 3 have the same fluorescent ion and crystal structure as those used in example 1. Thus, the 1/10 afterglow time of the phosphors used in example 2 and example 3 is similar to that of the phosphor used in example 1, is in the range of 100 µs to less than 1000 µs, and is estimated to be at least 10 µs or more.

### (Fluorescence property in excitation by laser beam)

Next, wavelength converters including phosphors used in examples 1 to 3 (wavelength converters in example 1 to 3) were prepared, and the fluorescence property was evaluated.

Specifically, the wavelength converter according to example 1 was manufactured by the following procedure. First, a phosphor paste was prepared by mixing the phosphor powder used in example 1 and a sealing material (polysilsesquioxane manufactured by Konishi Chemical Ind. Co., Ltd.) using a mortar and pestle so that the filling ratio of the phosphor was 40 vol%.

Next, a sapphire substrate (length 9 mm, width 9 mm, thickness 0.5 mm) having a dichroic mirror on one side and an anti-reflection coating (AR coating) on the other side was prepared. Then, the above-described phosphor paste was applied to the surface of the dichroic mirror on the sapphire substrate by a screen printing method. The mesh printing plate used for the screen printing was a circular plate having a mesh opening of 74 µm (200 mesh) and a diameter of 7.8 mm. Thereafter, the sapphire substrate applied with the phosphor paste was subjected to a heat treatment at 200 °C for 2 hours to harden the sealing material, thereby obtaining the wavelength converter of example 1.

Wavelength converters of examples 2 and 3 made from ceramic molded bodies were prepared by the following procedure. First, the top and bottom surfaces of each ceramic molded body (cylindrical shape) were polished using a tabletop polishing machine. The thicknesses of the ceramic molded bodies after polishing were 400 µm (example 2) and 300 µm (example 3). Then, the side surface of each ceramic molded body was polished using the same polishing machine to obtain a molded body having a regular square prism shape. The upper surface and the bottom surface of the molded body are square having a side length of 7 mm.

Next, the molded body after polishing was attached to a sapphire substrate (length 9 mm, width 9 mm, thickness 0.5 mm). Note that the sapphire substrate used has a dichroic mirror on one side and an anti-reflection coating (AR coating) on the other side. Then, 0.04 mL of polysilazane (liquid) was dropped onto the surface of the sapphire substrate on which the dichroic mirror was disposed, and the molded body after polishing was placed thereon and heated at 90 °C for 6 hours to attach the molded body on the sapphire substrate. Thus, the wavelength converters of examples 2 and 3 were obtained.

The obtained wavelength converter of each example was placed at the center of the integrating sphere. Next, blue laser beam emitted by a solid-state light emitting element installed outside the integrating sphere was guided to the center of the integrating sphere by using a light guide member to be emitted to the wavelength converter. That is, the wavelength converter was placed at the light output end of the light guide member, and the wavelength converter was excited by the blue laser beam (wavelength 450 nm) emitted by the solid-state light emitting element. Then, the fluorescence spectrum of the fluorescence emitted by the wavelength converter was measured by a multichannel spectrometer.

In the excitation of the wavelength converter of example 1, the blue laser beam was converted into a blue pulsed laser beam having a frequency of 100 Hz and a duty ratio of 1% using a function generator. The energy density of the blue pulsed laser beam emitted to the wavelength converter of example 1 was set to 3.15 W/mm². Note that the energy density of the blue pulsed laser beam is the energy density during irradiation of the wavelength converter with the blue pulsed laser beam.

In the excitation of the wavelength converters of example 2 and example 3, the blue laser beam was converted into a blue pulsed laser beam having a frequency of 1000 Hz and a duty ratio of 20% using a function generator. The energy of the blue pulsed laser beam emitted to the wavelength converters of example 2 and example 3 was both set to 6.85 W. Note that the energy of the blue pulsed laser beam is the energy during irradiation of the wavelength converters with the blue pulsed laser beam.

Fig. 7 illustrates the fluorescence spectrum of the wavelength converter of example 1. In Fig. 7, the fluorescence spectrum is normalized to have a maximum intensity value of 1. The fluorescence spectrum of the wavelength converter of example 1 was a broad spectrum determined to be attributed to the d-d transition of Cr³⁺. In the wavelength converter of example 1, the wavelength at which the intensity of the fluorescence spectrum becomes maximum was 730 nm. That is, the fluorescence spectrum of the wavelength converter of example 1 had a maximum intensity value in a wavelength range exceeding 710 nm. The fluorescence spectrum of the wavelength converter of example 1 had a light component over the entire wavelength range of 700 nm to 800 nm.

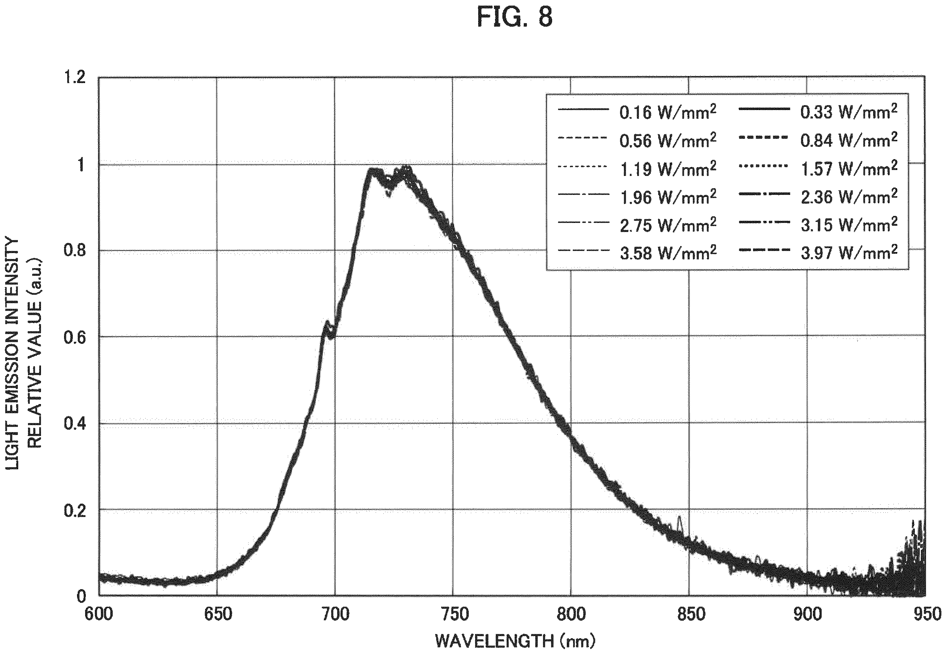

Fig. 8 illustrates fluorescence spectra of the wavelength converter of example 1 when the energy density of the blue pulsed laser beam is changed. The energy density of the blue pulsed laser beam was varied from 0.16 W/mm² to 3.97 W/mm². Note that in Fig. 8, the fluorescence spectra are normalized to have a maximum intensity value of 1. As is seen from Fig. 8, the fluorescence spectra of the wavelength converter of example 1 did not show any spectral shift or spectral shape change even when the energy density of the blue pulsed laser beam was changed.

Further, Table 1 shows the energy of a wavelength-converted light (fluorescence) emitted from the wavelength converter of example 1 when the wavelength converter is excited by a blue pulsed laser beam having different energy densities. As is seen from Table 1, when the energy density of the blue pulsed laser beam was increased from 0.16 W/mm² to 3.97 W/mm², the energy of the wavelength-converted light increased substantially in proportion to the energy density. That is, it was found that the Cr³⁺-activated phosphor maintains a high light emission efficiency even in a range where the energy density of the excitation light is high. This result is surprisingly different from the conventional common knowledge in which the use of a short afterglow (less than 10 µs) phosphor is indispensable to prevent the saturation of fluorescence output.

**[Table 1]**

| Energy density of blue pulsed laser beam (W/mm²) | Energy of wavelength-converted light (W) |
|---|---|
| 0.16 | 0.004 |
| 0.33 | 0.007 |
| 0.56 | 0.011 |
| 0.84 | 0.015 |
| 1.19 | 0.02 |
| 1.57 | 0.025 |
| 1.96 | 0.03 |
| 2.36 | 0.035 |
| 2.75 | 0.04 |
| 3.15 | 0.044 |
| 3.58 | 0.049 |
| 3.97 | 0.054 |

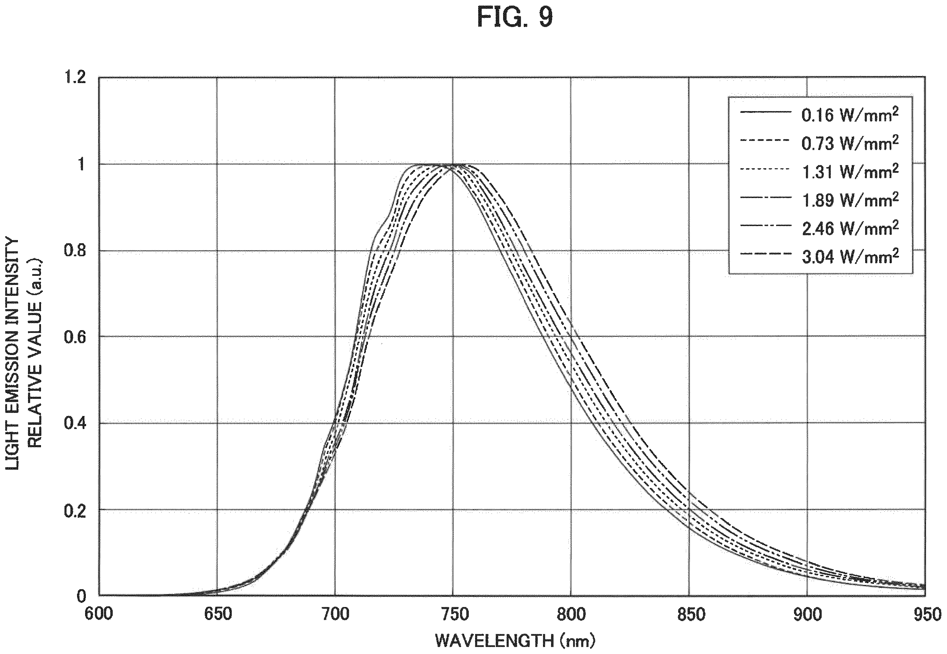

For reference, Fig. 9 shows fluorescence spectra of the wavelength converter of example 1 when it was excited by a blue continuous laser beam having different energy densities. Here, as the wavelength converter, the above-described raw material for calcining formed into a pellet by using a hand press was calcined in air at 1450 °C for 1 hour. The energy density of the blue laser beam emitted to the wavelength converter was varied from 0.16 W/mm² to 3.04 W/mm². Note that in Fig. 9, the fluorescence spectra are normalized to have a maximum intensity value of 1.

As is seen from Fig. 9, when the wavelength converter of example 1 was excited by a blue continuous laser beam, the fluorescence spectra of the wavelength converter shifted to the longer wavelength side with an increase in energy density. Specifically, when the energy densities of the blue laser beam were 0.16 W/mm², 0.73 W/mm², 1.31 W/mm², 1.89 W/mm², 2.46 W/mm², and 3.04 W/mm², the peak wavelengths of the fluorescence spectra of the wavelength converter were 741 nm, 743 nm, 746 nm, 749 nm, 752 nm, and 755 nm, respectively.

Table 2 shows, for reference, the energy of the wavelength-converted light (fluorescence) emitted from the wavelength converter of example 1 and the temperature of the wavelength converter of example 1 when the wavelength converter of example 1 is excited by a blue continuous laser beam having different energy densities. Note that the temperature of the wavelength converter of example 1 was measured using a thermographic camera. As shown in Table 2, the temperature of the wavelength converter of example 1 increased as the energy density of the blue continuous laser beam increased. Thus, it was found that the light emitting device that excites the wavelength converter of example 1 provided at the light output end of the light guide member by the continuous light having a high energy density may cause burns or the like and is difficult to be used for endoscopic applications.

**[Table 2]**

| Energy density of blue continuous laser beam (W/mm²) | Energy of wavelength-converted light (W) | Temperature of wavelength converter (°C) |
|---|---|---|
| 0.16 | 0.09 | 27 |
| 0.73 | 0.25 | 41 |
| 1.31 | 0.42 | 59 |
| 1.89 | 0.59 | 80 |
| 2.46 | 0.74 | 102 |
| 3.04 | 0.88 | 130 |

As is seen from Table 2, even when the energy density of the blue continuous laser beam was increased from 0.16 W/mm² to 3.04 W/mm², the wavelength converter of example 1 emitted light of high energy. That is, it was found that the Cr³⁺-activated phosphor maintains a high light emission efficiency even in a range where the energy of the excitation light is high. This result is surprisingly different from the conventional common knowledge in which the use of a short afterglow (less than 10 µs) phosphor is indispensable to prevent the saturation of fluorescence output. Considering that when the phosphor is excited by a high energy laser beam, the phosphor generates heat and the temperature of the phosphor rises, it is also said that the phosphor of example 1 is an excellent phosphor with little decrease in light emission efficiency due to temperature rise.

The results in Fig. 9 and Table 2 show that, when the wavelength converter of example 1 was excited by a continuous laser beam, the fluorescence spectrum of the wavelength-converted light shifted to the longer wavelength side with an increase in temperature of the wavelength converter. That is, it is said that the fluorescence spectrum of the Cr³⁺-activated phosphor shifts to the longer wavelength side as the temperature increases. In contrast, as illustrated in Fig. 8, when the wavelength converter of example 1 was excited by the pulsed laser beam, no spectral shift of the wavelength-converted light was observed even when the energy density was increased. Thus, it is said that the temperature of the wavelength converter of example 1 is hardly increased by using the configuration of exciting the wavelength converter by the pulsed laser beam.

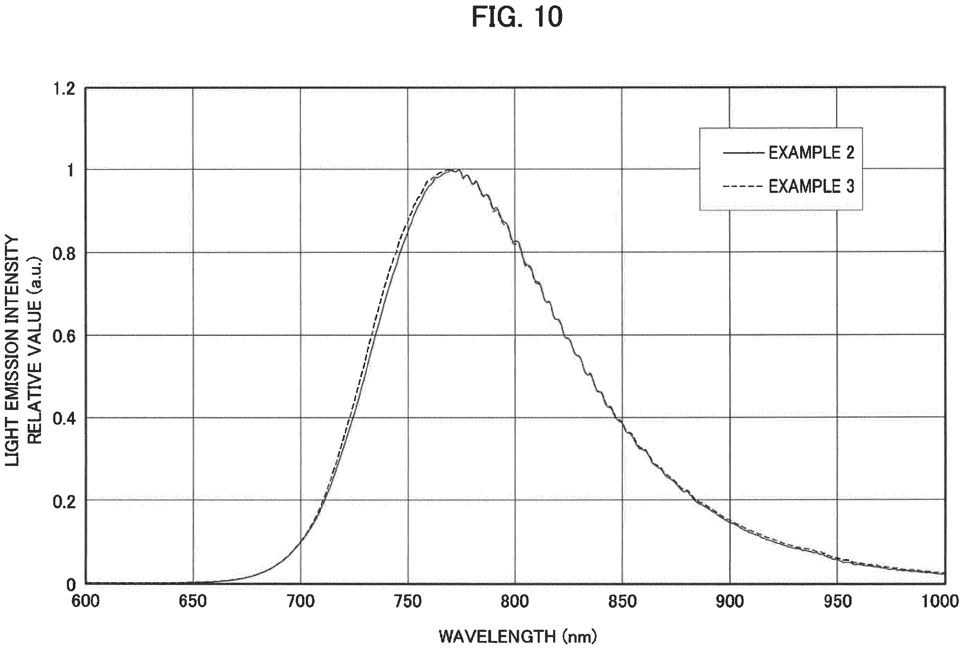

Fig. 10 illustrates the fluorescence spectra of the wavelength converters of example 2 and example 3. Also in Fig. 10, the fluorescence spectra are normalized to have a maximum intensity value of 1. The fluorescence spectra of the wavelength converters of examples 2 and 3 were broad spectra determined to be attributed to the d-d transition of Cr³⁺. In the wavelength converters of examples 2 and 3, the wavelengths at which the intensity of the fluorescence spectrum becomes maximum were 774 nm and 770 nm, respectively. That is, the fluorescence spectra of the wavelength converters of examples 2 and 3 each had a maximum intensity value in a wavelength range exceeding 710 nm. The fluorescence spectra of the wavelength converters of examples 2 and 3 each had a light component over the entire wavelength range from 700 nm to 800 nm.

Table 3 shows energies of wavelength-converted lights (fluorescence) emitted from the wavelength converters of examples 2 and 3 and the energy conversion efficiencies of the wavelength converters of examples 2 and 3 when the wavelength converters are excited by a blue pulsed laser beam with an energy of 6.85 W. As is seen from Table 3, the energies of the wavelength-converted lights (fluorescence) emitted from the wavelength converters of examples 2 and 3 were 1.45 W and 1.70 W, respectively, and both of them had high outputs of 1 W or more. This result shows that using the wavelength converter of the present example provides a high-power near-infrared light emitting device emitting a near-infrared light having an energy of 1 W or more.

The energy conversion efficiencies of the wavelength converters of examples 2 and 3 were 21.2% and 24.8%, respectively, which were above 20%.

**[Table 3]**

| | Energy of excitation light (W) | Energy of wavelength-converted light (W) | Energy conversion efficiency (%) |
|---|---|---|---|
| Example 2 | 6.85 | 1.45 | 21.2 |
| Example 3 | 6.85 | 1.70 | 24.8 |

When the wavelength converters of examples 2 and 3 were excited by a blue pulsed laser beam with an energy of 6.85 W, the fluorescent spot areas of fluorescence emitted by the wavelength converters were both about 7.0 mm², which was smaller than 10 mm². When the energies per unit area of fluorescence emitted from the wavelength converters of examples 2 and 3 were calculated, they were 0.21 W/mm² and 0.24 W/mm², respectively, which exceeded 0.1 W/mm² and also exceeded 0.2 W/mm². This result shows that the wavelength converters of examples 2 and 3 emit a near-infrared light with an extremely large light energy density. Thus, the wavelength converter according to the present embodiment makes it possible to provide a light emitting device that functions as a point light source emitting a near-infrared high output light, which has not existed before.

The above-described results also show that the light emitting device according to the present embodiment is a high output near-infrared light emitting device that is usable in an environment where the temperature is limited because the temperature of the light output end of the light guide member is hardly increased.

Although the contents of the present embodiment have been described above in accordance with the examples, it is obvious to those skilled in the art that the present embodiment is not limited to these descriptions and that various modifications and improvements are possible.

The entire contents of Japanese Patent Application No. 2019-094624 (filed on: May 20, 2019) are incorporated herein by reference.

### INDUSTRIAL APPLICABILITY

According to the present disclosure, there are provided a light emitting device capable of emitting a near-infrared light, which is usable even in a temperature-limited environment, such as inside a living body, and an electronic apparatus and an inspection method using the light emitting device.

### REFERENCE SIGNS LIST

- 1, 1A: Light emitting device
- 2: Excitation light source (solid-state light emitting element)
- 3: Light guide member
- 3a: Light input end
- 3b: Light output end
- 4, 4A: Wavelength converter
- 5: First phosphor
- 7: Primary light (pulsed light)
- 8: First wavelength-converted light (wavelength-converted light)

## Claims

1. A light emitting device, comprising:
a solid-state light emitting element that emits a pulsed light;
a wavelength converter comprising a phosphor that emits a wavelength-converted light having a wavelength longer than that of the pulsed light; and
a light guide member comprising a light input end and a light output end,
wherein the wavelength converter is disposed on a light output end side of the light guide member,
the pulsed light is input to the light guide member through the light input end and output through the light output end to be emitted to the wavelength converter, and
the wavelength-converted light has a fluorescence spectrum having a maximum intensity value in a wavelength range exceeding 710 nm.

2. The light emitting device according to claim 1, wherein the pulsed light has a frequency of 10 Hz or more and a duty ratio of 50% or less.

3. The light emitting device according to claim 1 or 2, wherein the pulsed light is an ultraviolet light or a visible light.

4. The light emitting device according to any one of claims 1 to 3, wherein the phosphor comprises a Cr³⁺-activated phosphor.

5. The light emitting device according to any one of claims 1 to 4, wherein the wavelength-converted light emitted by the phosphor has a 1/10 afterglow time of 10 µs or more.

6. The light emitting device according to any one of claims 1 to 5, wherein the wavelength-converted light has an energy of 1 W or more.

7. The light emitting device according to any one of claims 1 to 6, wherein the phosphor is a ceramic molded body.

8. The light emitting device according to any one of claims 1 to 7, further comprising a lens disposed near a surface of the wavelength converter, the surface opposite to a surface facing the light output end of the light guide member, the lens condensing the wavelength-converted light.

9. The light emitting device according to any one of claims 1 to 8, the light emitting device is a medical light emitting device usable in a fluorescence imaging method or a photodynamic therapy.

10. The light emitting device according to any one of claims 1 to 8, the light emitting device is a light source for a sensing system or a lighting system for a sensing system.

11. An electronic apparatus, comprising the light emitting device according to any one of claims 1 to 8.

12. The electronic apparatus according to claim 11, wherein the electronic apparatus is any of an information recognition device, a sorting device, a detection device, or an inspection device.

13. The electronic apparatus according to claim 12, wherein the inspection device is any of a medical inspection device, an agricultural and livestock industry inspection device, a fishery inspection device, or an industrial inspection device.

14. An inspection method, comprising:
using the light emitting device according to any one of claims 1 to 8.
